(19) **Europäisches Patentamt / European Patent Office / Office européen des brevets**

(11) **EP 4 686 717 A1**

(12) **EUROPEAN PATENT APPLICATION**

(43) Date of publication:
04.02.2026 Bulletin 2026/06

(21) Application number: 24191864.8

(22) Date of filing: 30.07.2024

(51) International Patent Classification (IPC):
$C07C\ 29/80^{(2006.01)}$     $C07C\ 29/151^{(2006.01)}$
$C07C\ 31/04^{(2006.01)}$     $C25B\ 1/04^{(2021.01)}$
$C25B\ 1/23^{(2021.01)}$     $C01B\ 13/02^{(2006.01)}$

(52) Cooperative Patent Classification (CPC):
(C-Sets available)
C07C 29/1518; C07C 29/80; C25B 1/042;
C25B 1/23; C25B 15/021; C25B 15/081     (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **CASALE SA**
**6900 Lugano (CH)**

(72) Inventors:
- **MOREO, Pietro**
  **6900 Lugano (CH)**
- **GENOVA, Giovanni**
  **22100 Como (IT)**
- **MUSCIONICO, Isabella**
  **22030 Orsenigo (CO) (IT)**

(74) Representative: **M. Zardi & Co S.A.**
**Via G. B. Pioda, 6**
**6900 Lugano (CH)**

(54) **GREEN METHANOL PROCESS AND PLANT**

(57)     A process or plant for the synthesis of methanol (MeOH). The process comprises:
(a) passing a water-containing stream (3) through an electrolysis unit (4) to produce a cathode-side stream (5) comprising hydrogen ($H_2$) and an anode-side stream (6) comprising oxygen ($O_2$);
(b) heat-exchanging said cathode-side stream (5) and optionally said anode-side stream (6) in one or more indirect heat exchanger(s) (7, 8, 32, 33) to obtain a cathode-side heat-exchanged stream (9) and optionally an anode-side heat-exchanged stream (10);
(c) condensing said cathode-side heat-exchanged stream (9) to separate a liquid condensate product (11) and a syngas (12);
said cathode-side stream (5) and/or said syngas (12) comprise carbon dioxide ($CO_2$) and optional carbon monoxide (CO) added through a separate stream (2);
(d) compressing said syngas (12) and then feeding compressed syngas (13) to a MeOH synthesis loop (14) wherein catalytic conversion of said compressed syngas (13) into MeOH is carried out under methanol synthesis conditions, thus obtaining a crude methanol stream (15);
(e) distilling said crude methanol stream (15) in one or more distillation column(s) (16, 17) to give a refined MeOH product (22);
wherein said one or more indirect heat exchanger(s) (7, 8, 32, 33) provide a heat input to said one or more distillation column(s) (16, 17), and/or to said MeOH synthesis loop (14), and/or to said electrolysis unit (4).

Fig. 1.A

**(Cont. next page)**

EP 4 686 717 A1

(52) Cooperative Patent Classification (CPC): (Cont.)

C-Sets
**C07C 29/1518, C07C 31/04;**
**C07C 29/80, C07C 31/04**

## Description

### Field of application

The invention is in the field of methanol (MeOH) production. The invention particularly pertains to a process and a plant for the synthesis of methanol.

### Prior art

At present, synthesis gas (syngas) required for methanol synthesis is produced by reformation of natural gas in approx. 65% of the existing plants, while the remaining part is based on coal gasification. One of the main drawbacks of the current plants is that the involved processes are highly carbon intensive, i.e., they produce high emissions of carbon dioxide ($CO_2$).

Growing concerns about global climate changes make it urgent to investigate alternative pathways to produce methanol.

Among others, the so-called "green methanol" synthesis pathway entails the use of renewable energy to produce hydrogen ($H_2$) and of $CO_2$ from sustainable process or recovery (e.g., from flue gases or Direct Air Capture).

Syngas obtained through existing electrolysis technologies - such as alkaline water electrolysis or proton exchange membrane electrolysis - has a composition that does not conform exactly to methanol synthesis due to high amounts of carbon dioxide and a substantial lack of carbon monoxide (CO). A high concentration of $CO_2$ in the syngas results in corresponding high amounts of water produced due to the reaction stoichiometry, thus reducing the reaction yield to give methanol and reducing the expected lifetime of the synthesis catalyst.

In view of these considerations, it is highly desirable to develop an energyefficient, environmentally friendly, and economically viable process and plant for the synthesis of methanol on an industrial scale.

### Summary of the invention

The aim of the present invention is to solve the drawbacks of the prior art.

The invention is based on the judicious insight that methanol synthesis produces one or more electrolysis effluent(s) whose calorific content can be exploited within the methanol synthesis process and plant, making said process more efficient.

More precisely, the effluents from the electrolysis unit (e.g. comprising SOECs), in view of their high temperature, contain a high amount of heat that can be reused in the methanol synthesis process and/or in the electrolysis itself.

Accordingly, one aspect of the present invention is a process, preferably a thermally integrated process, for the synthesis of methanol (MeOH) comprising the following steps:

(a) passing a water-containing stream through an electrolysis unit to produce a cathode-side stream comprising hydrogen ($H_2$) and an anode-side stream comprising oxygen ($O_2$);

(b) heat-exchanging said cathode-side stream and optionally said anode-side stream in one or more indirect heat exchanger(s) to obtain a cathode-side heat-exchanged stream and optionally an anode-side heat-exchanged stream;

(c) condensing said cathode-side heat-exchanged stream to separate a liquid condensate product and a syngas;
said cathode-side stream and/or said syngas comprise carbon dioxide ($CO_2$) and optional carbon monoxide added through a separate stream;

(d) compressing said syngas and then feeding compressed syngas to a MeOH synthesis loop wherein catalytic conversion of said syngas into MeOH is carried out under methanol synthesis conditions, thus obtaining a crude methanol stream;

(e) distilling said crude methanol stream in one or more distillation column(s) to give a refined MeOH product;

wherein said one or more indirect heat exchanger(s) provide a heat input to said one or more distillation column(s), and/or to said MeOH synthesis loop, and/or to said electrolysis unit.

A further aspect of the present invention is a plant for producing methanol comprising:

- an electrolysis unit fed with a water-containing stream and arranged to produce a cathode-side stream comprising hydrogen ($H_2$) and an anode-side stream comprising oxygen ($O_2$);

- one or more indirect heat exchanger(s) in a heat-exchanging relationship with said cathode-side stream and optionally said anode-side stream to obtain a cathode-side heat-exchanged stream and optionally an anode-side heat-exchanged stream;

- a condenser of said cathode-side heat-exchanged stream to separate a liquid condensate product and a syngas;

- a line for adding carbon dioxide ($CO_2$) and optional carbon monoxide (CO) to said cathode-side stream and/or said syngas through a separate stream;

- at least one compressor for compressing said syngas and at least a MeOH synthesis loop fed with compressed syngas and arranged for a catalytic conversion of said syngas into MeOH under methanol synthesis conditions, thus obtaining a crude methanol stream;

- one or more distillation column(s) for distilling the crude methanol stream to give a refined MeOH product;

wherein said one or more indirect heat exchanger(s) provide a heat input to said one or more distillation column(s), and/or to said MeOH synthesis loop, and/or to said electrolysis unit.

Advantageous effects of the invention

Advantageously, methanol synthesis can be performed with a co-electrolysis of water and $CO_2$, preferably using solid-oxide electrolysis cells (SOECs).
The expression "co-electrolysis" means that electrolysis of $H_2O$ and $CO_2$ is performed in the same electrolysis unit (see, e.g., unit 4 in the figures) at the same time.
On one side, co-electrolysis provides stoichiometrically suitable amounts of carbon monoxide, carbon dioxide and hydrogen in the syngas for an improved MeOH synthesis.
According to another advantageous aspect, a $H_2O/CO_2$ co-electrolysis allows to feed water (steam) and carbon dioxide to the electrolysis unit thus producing - on a cathode side - a syngas comprising steam, CO, $CO_2$ and $H_2$, basically converting $CO_2$ into CO while producing $H_2$ and - on an anode side - a stream of oxygen ($O_2$) by electrolysis in the same stack.
Advantageously, the process of the present invention provides a heat integration that can reduce the global energy consumption up to 30%.
Advantageously, the process of the present invention can be implemented in existing plants, replacing partially or totally the existing syngas generation section, without changing the methanol synthesis loop drastically.
Advantageously, since SOECs and distillation both involve energy demanding processes, heat integration achieved by the present process and plant is beneficial to at least in part satisfy energy requirements and lowering global energy consumption.
Advantageously, the products of co-electrolysis are produced at high temperatures, so that their residual heat can be partially recovered by the feed effluent(s) and/or can be partially used to produce steam under pressure. This recovered steam may be used within the SOECs, or exported to other units of the plant to further lower energy consumption, or can be exported from the plant, e.g. to a $CO_2$ capture unit such as a stripper reboiler of a carbon dioxide recovery.
Advantageously, the process and plant of the present invention allow to achieve a reduction of the total number and/or size of installed equipment either inside battery limits (ISBL) or outside battery limits (OSBL).

Still a further advantage is that if the $CO_2$ source is biogenic, thus it contains methane, such component is converted into additional syngas, through a reforming reaction in the SOEC, instead of being fed as inert compound to the methanol synthesis loop.
Advantageously, the process and plant of the present invention enable an improvement or optimization of the composition of the produced syngas in terms of CO, $CO_2$ and $H_2$ ratio since it enables to set the stoichiometric number M in the here described range.

Description of the preferred embodiments

Preferably, said electrolysis unit 4 comprises solid-oxide electrolysis cells (SOECs).
According to different embodiments, said stream 2 may be a flue gas, an industrial waste gas, a stream of air-captured $CO_2$, and/or a stream of biogenic $CO_2$.
According to a preferred embodiment, said cathode-side stream 5 is obtained by passing said water-containing stream 3 and said stream 2 through said electrolysis unit 4. In this embodiment, said electrolysis unit 4 is a co-electrolysis unit of the water-containing stream 3 and of said stream 2.
According to another embodiment, said heat input is a direct heat input.
Preferably, said cathode-side stream 5 and/or said anode-side stream 6 exchange heat with a process fluid or process fluids of said one or more distillation column(s) 16, 17, and/or of said MeOH synthesis loop 14, and/or of said electrolysis unit 4 in said one or more indirect heat exchanger(s) 7, 8, 32, 33.
In the present description "direct" means that there is no (intermediate) heat-exchanging medium/media that transfers heat from the cathode-side stream 5 and/or said anode-side stream 6 to said process fluid or process fluids.
According to still another embodiment, said heat input is an indirect heat input.
Preferably, said cathode-side stream 5 and/or said anode-side stream 6 exchange heat with one or more heat-exchanging medium/media (such as water or steam) in said one or more indirect heat exchanger(s) 7, 8. Said one or more heat-exchanging medium/media further exchange(s) heat with a process fluid or process fluids of said one or more distillation column(s) 16, 17, and/or of said MeOH synthesis loop 14, and/or of said electrolysis unit 4 in one or more further indirect heat exchanger(s).
According to still another embodiment, said one or more distillation column(s) 16, 17 is at least a topping column 16 comprising at least a bottom boiler.
Preferably, said bottom boiler is said one or more indirect heat exchanger(s) 7, 8, or is in a heat-exchanging relationship with said one or more indirect heat exchanger(s) 7, 8, for providing said heat input to said topping column 16.

More preferably, said process comprises:
(f) feeding said crude methanol stream 15 to the topping column 16 to separate volatile compounds 18, such as $H_2$, CO, $CO_2$, and a bottom MeOH solution 19 as a refined MeOH product or topped MeOH product from said crude methanol stream 15.

According to a further embodiment, said one or more distillation column(s) 16, 17 is at least a refining column 17 comprising at least a bottom boiler. Preferably, said distillation columns 16, 17 may be more than two, e.g. from two to four refining columns 17.

Preferably, said bottom boiler is said one or more indirect heat exchanger(s) 7, 8, or is in a heat-exchanging relationship with said one or more indirect heat exchanger(s) 7, 8, for providing said heat input to said refining column 17.

Even more preferably, said process comprises a topping column 16 and a downstream refining column 17; and said process comprises:

(g) feeding said bottom MeOH solution 19 separated in the topping column 16 to the refining column 17; and

(h) distilling said bottom MeOH solution 19 to obtain a bottom water stream 20, a stream of fusel oil 21, and a MeOH stream 22 as a refined MeOH product.

In the present description "fusel oil" means a mixture of heavier compounds comprising water, higher alcohols, methanol, and alkanes.

According to further embodiments, said one or more indirect heat exchanger(s) 7, 8 provide said heat input to a boiler feed water which is used as cooling medium in heat-exchange bodies 23, 24 of an isothermal catalytic converter of the MeOH synthesis loop 14 and/or of the electrolysis unit 4.

According to further embodiments, said one or more indirect heat exchanger(s) 7, 8 provide said heat input to steam produced in heat-exchange bodies 23, 24 of an isothermal catalytic converter of the MeOH synthesis loop 14 and/or of the electrolysis unit 4.

According to another embodiment, at least one of said one or more indirect heat exchanger(s) 32, 33 is at least a feed-effluent heat exchanger of the electrolysis unit 4.

The MeOH synthesis loop preferably comprises a catalytic converter provided with a fixed bed that operates in the pressure range comprised from 50 bar to 120 bar and in a temperature range comprised from 200 °C to 300 °C.

Said electrolysis unit 4 or said SOECs is/are preferably powered by a renewable energy source 25.

SOECs are more preferably operated in exothermic mode.

Preferably, said syngas 12 has a molar ratio M comprised from 1.90 to 2.15, preferably from 1.95 to 2.10, such ratio M being defined as:

$$M = (H_2 - CO_2)/(CO + CO_2)$$

According to an embodiment of the plant, said one or more distillation column(s) 16, 17 is at least a topping column 16 and/or at least a refining column 17 comprising at least a bottom boiler.

Preferably, said bottom boiler is said one or more indirect heat exchanger(s) 7, 8, or is in a heat-exchanging relationship with said one or more indirect heat exchanger(s) 7, 8, for providing said heat input to said at least one topping column 16 and/or at least one refining column 17.

The plant 1 preferably comprises at least one indirect heat exchanger 8 in a heat-exchanging relationship with said cathode-side stream 5 and at least one indirect heat exchanger 7 in a heat-exchanging relationship with said anode-side stream 6.

The plant preferably comprises a renewable energy source 25 powering said electrolysis unit (4) or solid-oxide electrolysis cells (SOECs).

The renewable energy source 25 preferably comprises one or more photovoltaic panel(s) and/or wind turbine(s) and/or water turbine(s).

Description of the figures

Figures from 1.A to 5 show schematic process layouts of a methanol plant according to preferred embodiments of the present invention.

Detailed description of the preferred embodiments

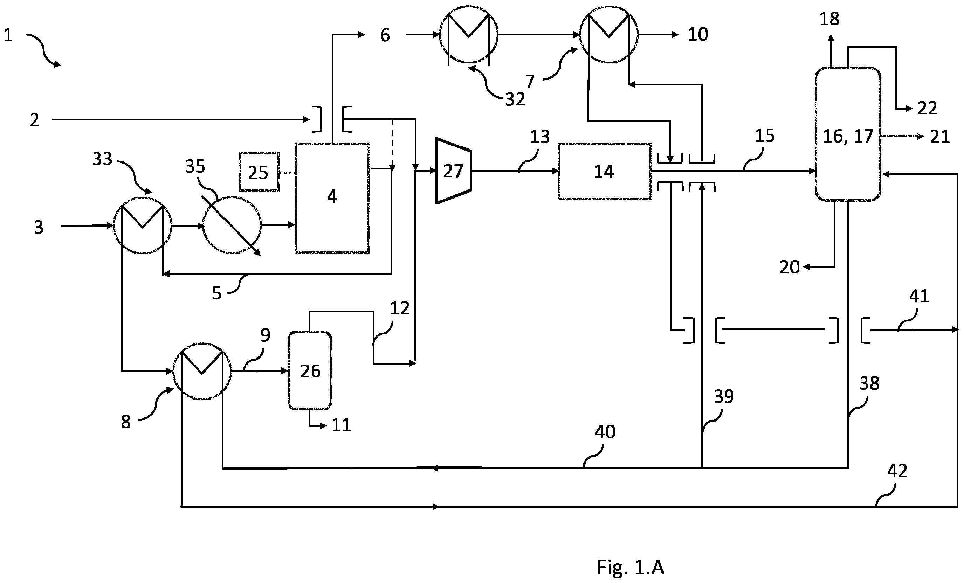

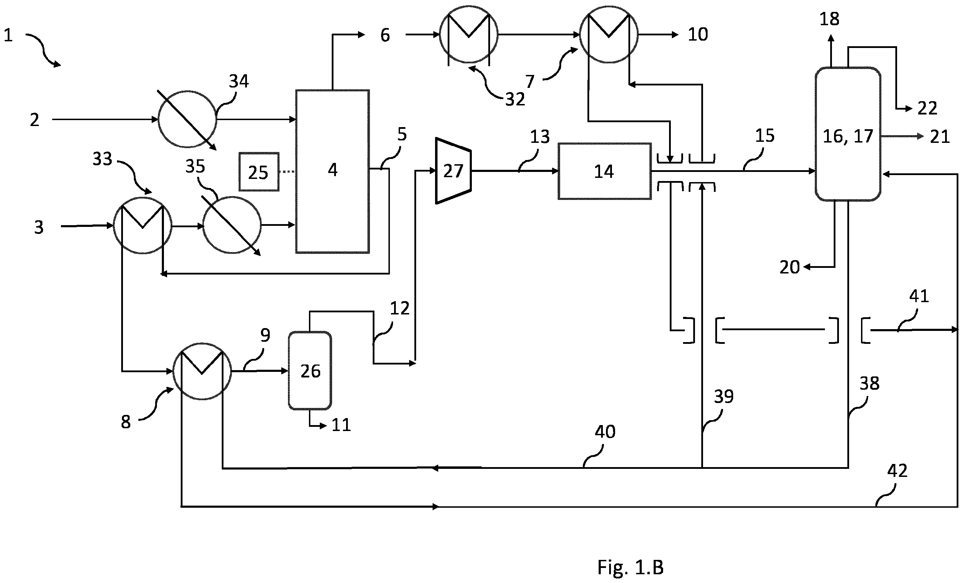

Fig. 1.A shows a plant 1 for the synthesis of methanol (MeOH) according to a first preferred embodiment of the present invention. Such plant 1 comprises an electrolysis unit 4, one or more indirect heat exchanger(s) 7, 8, a condenser 26, a compressor 27, a MeOH synthesis loop 14, and one or more distillation column(s) 16, 17.

A water-containing stream 3 is passed through a second heat exchanger 33 and then through a further second heat exchanger 35, and is then fed to the electrolysis unit 4.

The electrolysis unit 4 is powered by an electrical source (e.g., a renewable energy source 25) to produce - by electrolysis - a cathode-side stream 5 comprising hydrogen ($H_2$) and optional steam, and an anode-side stream 6 comprising oxygen $O_2$.

The anode-side stream 6 is heat exchanged in a first heat exchanger 32 and then in the indirect heat exchanger 7 so that an anode-side heat-exchanged stream 10 is obtained. This stream 10 can be exported from the plant.

The cathode-side stream 5 is cooled - by indirect heat exchange - in the second heat exchanger 33 with the water-containing stream 3, and then it goes through the indirect heat exchanger 8 so that a cathode-side heat-exchanged stream 9 is obtained.

The cathode-side heat-exchanged stream 9 is then fed to a condenser 26, wherein it is separated in a liquid condensate product 11 containing condensed steam and a syngas 12.

Carbon dioxide ($CO_2$) and optional carbon monoxide (CO) is/are added to said cathode-side stream 5 and/or said syngas 12 through a separate stream 2.

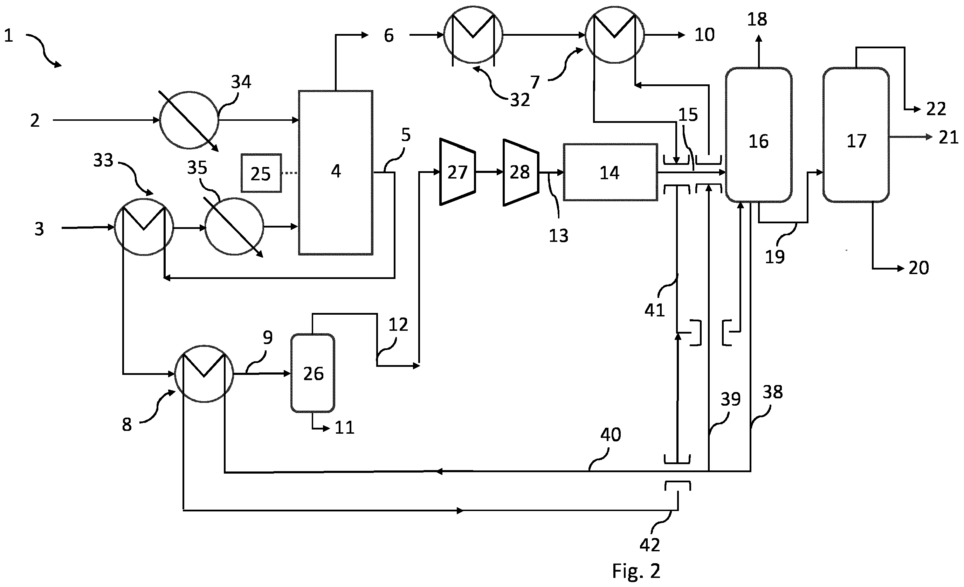

Said syngas 12 is compressed in a compressor 27 to obtain a compressed syngas 13. Multistage compression in two or more compressors 27, 28 - see e.g. Fig. 2 - may be used to obtain said compressed syngas 13.

The compressed syngas 13 is then fed to the MeOH synthesis loop 14 wherein catalytic conversion of said compressed syngas 13 into MeOH is carried out under methanol synthesis conditions. A crude methanol stream 15 is thus obtained as an effluent of the MeOH synthesis loop 14.

Said crude methanol stream 15 is distilled in one or more distillation column(s) 16, 17 to give a refined MeOH product 22. Distillation in said one or more distillation column(s) 16, 17 may provide also a stream of volatile compounds 18, a stream of fusel oil 21, and a bottom water stream 20.

In this embodiment, said one or more indirect heat exchanger(s) 7, 8 provide a heat input to said one or more distillation column(s) 16, 17, either a direct or an indirect heat input.

More precisely, said one or more distillation column(s) 16, 17 may comprise at least a bottom boiler. Said bottom boiler may be embodied by said one or more indirect heat exchanger(s) 7, 8, or it may be in a heat-exchanging relationship with said one or more indirect heat exchanger(s) 7, 8, for providing said heat input to said distillation column(s) 16, 17. In particular, a bottom stream 38 is withdrawn from the distillation column(s) 16, 17 and sent to said one or more indirect heat exchanger(s) 7, 8 as one or more first stream(s) 39, 40 for exchanging heat with said cathode-side stream 5 and said anode-side stream 6. After heat exchange, one or more heat-exchanged first stream(s) 41, 42 are returned to the distillation column(s) 16, 17.

Fig. 1.B shows a plant 1 for the synthesis of methanol (MeOH) according to a second preferred embodiment of the present invention.

As a difference with respect to the embodiment of Fig. 1.A, a stream 2 containing $CO_2$ as main component is passed through a first heat exchanger 34 and is then fed to the electrolysis unit 4 together with the water-containing stream 3.

In the electrolysis unit 4, said cathode-side stream 5 is produced by co-electrolysis of the water-containing stream 3 and of said separate stream 2. The cathode-side stream 5 thus comprises steam, $CO_2$, CO, and $H_2$.

According to a preferred embodiment, said first heat exchanger 34 may be a feed-effluent heat exchanger, so that the heat exchangers numbered with 32 and 34 may be the same apparatus wherein said anode-side stream 6 and said $CO_2$-containing stream 2 exchange heat by indirect heat exchange.

Fig. 2 shows a plant 1 for the synthesis of methanol (MeOH) according to a third preferred embodiment of the present invention. This plant differs from the plant of Fig. 1.B in that the distillation columns comprise at least a topping column 16 and at least a refining column 17.

The crude methanol stream 15 is fed to the topping column 16 to separate volatile compounds 18 and a bottom MeOH solution 19 from said crude methanol stream 15. The bottom MeOH solution 19 is fed to the refining column 17, wherein said bottom MeOH solution 19 is distilled to obtain a bottom water stream 20, a stream of fusel oil 21, and a MeOH stream 22.

A bottom stream 38 is withdrawn from the topping column 16 and sent to said indirect heat exchangers 7, 8 as first streams 39, 40 for exchanging heat with said cathode-side stream 5 and said anode-side stream 6. After heat exchange, heat-exchanged first streams 41, 42 are returned to the topping column 16.

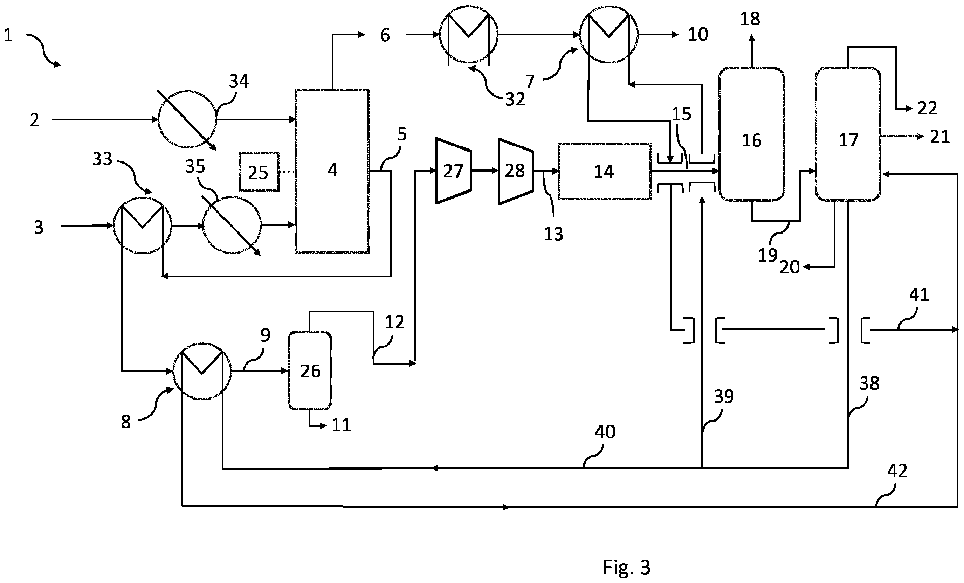

Fig. 3 shows a plant 1 for the synthesis of methanol (MeOH) according to a fourth preferred embodiment of the present invention. This plant differs from the plant of Fig. 2 in that the bottom stream 38 is withdrawn from the at least one refining column 17 and sent to said indirect heat exchangers 7, 8 as first streams 39, 40 for exchanging heat with said cathode-side stream 5 and said anode-side stream 6. After heat exchange, heat-exchanged first streams 41, 42 are returned to the refining column 17.

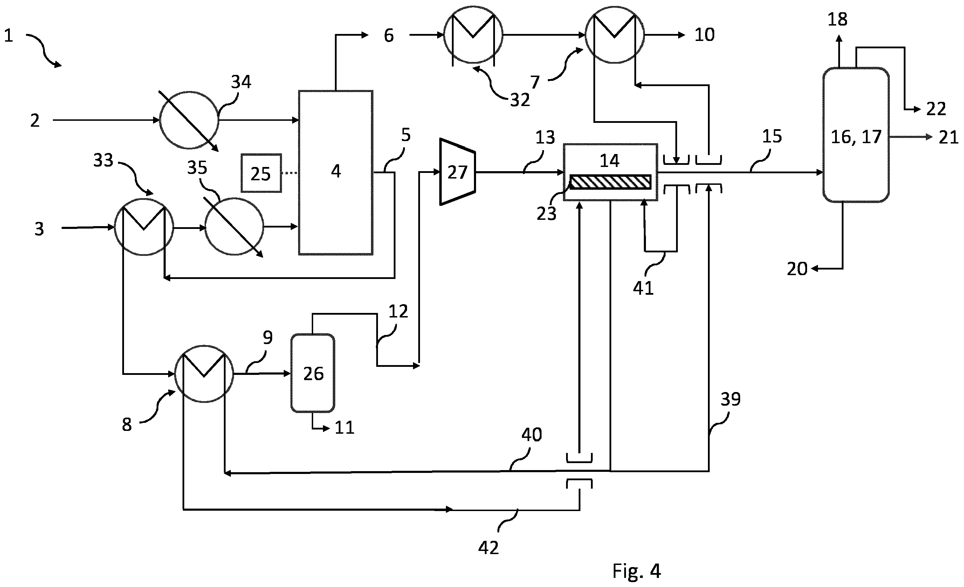

Fig. 4 shows a plant 1 for the synthesis of methanol (MeOH) according to a fifth preferred embodiment of the present invention. This plant differs from the plant of Fig. 1.B in that said one or more indirect heat exchanger(s) 7, 8 provide a heat input to the MeOH synthesis loop 14 and, in particular, to a boiler feed water which is used as cooling medium in an isothermal catalytic converter of the MeOH synthesis loop and/or to superheat steam produced by an isothermal catalytic converter of the MeOH synthesis loop. More precisely, said one or more indirect heat exchanger(s) 7, 8 provide said heat input the boiler feed water which is used as cooling medium to one or more heat-exchange bodies 23 of the catalytic converter and/or to superheat the steam produced by the isothermal catalytic converter of the MeOH synthesis loop.

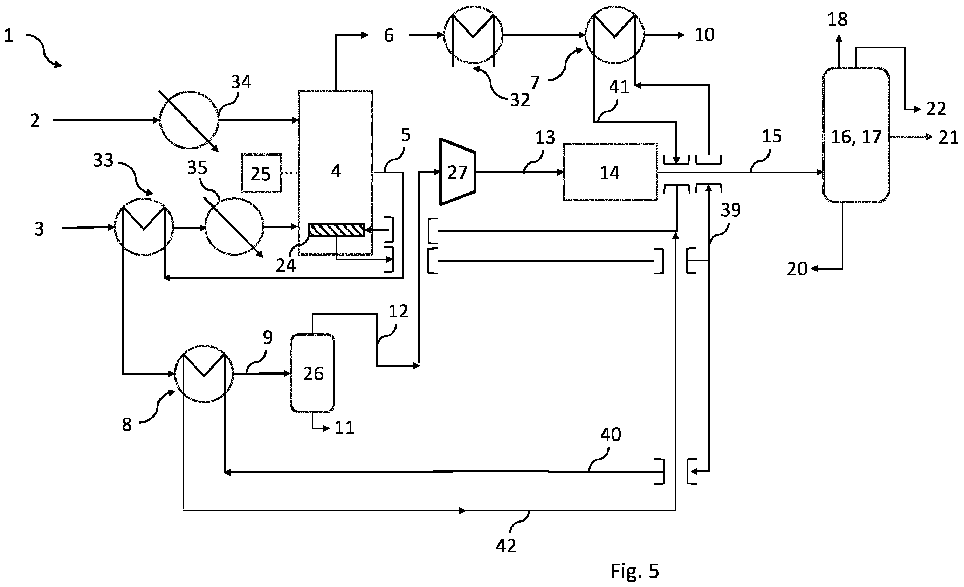

Fig. 5 shows a plant 1 for the synthesis of methanol (MeOH) according to a sixth preferred embodiment of the present invention. This plant differs from the plant of Fig. 1.B and of Fig. 4 in that said one or more indirect heat exchanger(s) 7, 8 provide a heat input to the electrolysis unit 4 and, in particular, to one or more heat-exchange bodies 24 of the electrolysis unit 4.

LIST OF THE REFERENCE SIGNS

| 1 | plant for the synthesis of methanol |
| 2 | stream containing carbon dioxide and optional carbon monoxide |
| 3 | water-containing stream, e.g. steam-containing stream |
| 4 | electrolysis unit, preferably co-electrolysis unit |
| 5 | cathode-side stream |
| 6 | anode-side stream |
| 7 | indirect heat exchanger |
| 8 | indirect heat exchanger |
| 9 | cathode-side heat-exchanged stream |
| 10 | anode-side heat-exchanged stream |
| 11 | liquid condensate product |
| 12 | syngas |
| 13 | compressed syngas |
| 14 | MeOH synthesis loop |
| 15 | crude methanol stream |
| 16 | distillation column, in particular topping column |
| 17 | distillation column, in particular refining column |
| 18 | volatile compounds |
| 19 | refined MeOH product, in particular bottom MeOH solution |
| 20 | bottom water stream |
| 21 | stream of fusel oil |
| 22 | refined MeOH product, in particular MeOH stream |
| 23 | heat-exchange bodies of the catalytic converter |
| 24 | heat-exchange bodies of the electrolysis unit |
| 25 | renewable energy source |
| 26 | condenser |
| 27 | compressor, preferably main compressor of the MeOH synthesis loop |
| 28 | compressor |
| 32 | first heat exchanger, preferably first feed-effluent heat exchanger |
| 33 | second heat exchanger, preferably second feed-effluent heat exchanger |
| 34 | first heat exchanger |
| 35 | second heat exchanger |
| 38 | bottom stream |
| 39 | first stream |
| 40 | first stream |
| 41 | heat-exchanged first stream |
| 42 | heat-exchanged first stream |

**Claims**

1. A process for the synthesis of methanol (MeOH) comprising the following steps:

   (a) passing a water-containing stream (3) through an electrolysis unit (4) to produce a cathode-side stream (5) comprising hydrogen ($H_2$) and an anode-side stream (6) comprising oxygen ($O_2$);

   (b) heat-exchanging said cathode-side stream (5) and optionally said anode-side stream (6) in one or more indirect heat exchanger(s) (7, 8, 32, 33) to obtain a cathode-side heat-exchanged stream (9) and optionally an anode-side heat-exchanged stream (10);

   (c) condensing said cathode-side heat-exchanged stream (9) to separate a liquid condensate product (11) and a syngas (12); said cathode-side stream (5) and/or said syngas (12) comprise carbon dioxide ($CO_2$) and optional carbon monoxide (CO) added through a separate stream (2);

   (d) compressing said syngas (12) and then feeding compressed syngas (13) to a MeOH synthesis loop (14) wherein catalytic conversion of said compressed syngas (13) into MeOH is carried out under methanol synthesis conditions, thus obtaining a crude methanol stream (15);

   (e) distilling said crude methanol stream (15) in one or more distillation column(s) (16, 17) to give a refined MeOH product (22);

   wherein said one or more indirect heat exchanger(s) (7, 8, 32, 33) provide a heat input to said one or more distillation column(s) (16, 17), and/or to said MeOH synthesis loop (14), and/or to said electrolysis unit (4).

2. The process according to claim 1, wherein said cathode-side stream (5) is obtained by passing said water-containing stream (3) and said stream (2) through said electrolysis unit (4); said electrolysis unit (4) being a co-electrolysis unit of the water-containing stream (3) and of said stream (2).

3. The process according to claim 1 or 2, wherein said heat input is a direct heat input, said cathode-side stream (5) and optionally said anode-side stream (6) exchanging heat with a process fluid or process fluids of said one or more distillation column(s) (16, 17), and/or of said MeOH synthesis loop (14), and/or of said electrolysis unit (4) in said one or more indirect heat exchanger(s) (7, 8).

4. The process according to claim 1 or 2, wherein said heat input is an indirect heat input, said cathode-side stream (5) and optionally said anode-side stream (6) exchanging heat with one or more heat-exchanging medium/media, such as water or steam, in said one or more indirect heat exchanger(s) (7, 8), and wherein said one or more heat-exchanging medium/media further exchange(s) heat with a process fluid or process fluids of said one or more distillation column(s) (16, 17), and/or of said MeOH synthesis loop (14) and/or of said electrolysis unit (4) in one or more further indirect heat exchanger(s).

5. The process according to any of the previous claims, wherein said one or more distillation column(s) (16, 17) is a topping column (16) comprising at least a bottom boiler; said bottom boiler being said one or more indirect heat exchanger(s) (7, 8), or being in a heat-exchanging relationship with said one or more indirect heat exchanger(s) (7, 8), for providing said heat input to said topping column (16); and wherein said process comprises:

   (f) feeding said crude methanol stream (15) to the topping column (16) to separate volatile compounds (18), such as $H_2$, CO, $CO_2$, and a bottom MeOH solution (19) as a topped MeOH product from said crude methanol stream (15).

6. The process according to any of the previous claims, wherein said one or more distillation column(s) (16, 17) is at least a refining column (17) comprising at least a bottom boiler; said bottom boiler being said one or more indirect heat exchanger(s) (7, 8), or being in a heat-exchanging relationship with said one or more indirect heat exchanger(s) (7, 8), for providing said heat input to said refining column (17).

7. The process according to claims 5 and 6, comprising a topping column (16) and a downstream refining column (17); and wherein said process comprises:

   (g) feeding said bottom MeOH solution (19) separated in the topping column (16) to the refining column (17); and
   (h) distilling said bottom MeOH solution (19) to obtain a bottom water stream (20), a stream of fusel oil (21), and a MeOH stream (22) as a refined MeOH product.

8. The process according to any of the previous claims, wherein said one or more indirect heat exchanger(s) (7, 8) provide said heat input to a boiler feed water which is used as cooling medium in heat-exchange bodies (23, 24) of an isothermal catalytic converter of the MeOH synthesis loop (14) and/or of the electrolysis unit (4).

9. The process according to any of the previous claims, wherein at least one of said one or more indirect heat exchanger(s) (32, 33) is at least a feed-effluent heat exchanger of the electrolysis unit (4).

10. The process according to any of the previous claims, wherein said electrolysis unit (4) comprises solid-oxide electrolysis cells (SOECs), said SOECs being powered by a renewable energy source (25) and operated in exothermic mode.

11. The process according to any of the previous claims, wherein said syngas (12) has a molar ratio M comprised from 1.90 to 2.15, preferably from 1.95 to 2.10, such ratio M being defined as:

$$M = (H_2 - CO_2)/(CO+CO_2)$$

12. A plant (1) for the synthesis of methanol (MeOH) comprising:

   - an electrolysis unit (4) fed with a water-containing stream (3) and arranged to produce a cathode-side stream (5) comprising hydrogen ($H_2$) and an anode-side stream (6) comprising oxygen ($O_2$);
   - one or more indirect heat exchanger(s) (7, 8, 32, 33) in a heat-exchanging relationship with said cathode-side stream (5) and optionally said anode-side stream (6) to obtain a cathode-side heat-exchanged (9) stream and optionally an anode-side heat-exchanged stream (10);
   - a condenser (26) of said cathode-side heat-exchanged stream (9) to separate a liquid condensate product (11) and a syngas (12);
   - a line for adding carbon dioxide ($CO_2$) and optional carbon monoxide (CO) to said cathode-side stream (5) and/or said syngas (12) through a separate stream (2);
   - at least one compressor (27, 28) for compressing said syngas (12) and at least a MeOH synthesis loop (14) fed with compressed syngas (13) and arranged for a catalytic conversion of said compressed syngas (13) into MeOH under methanol synthesis conditions, thus obtaining a crude methanol stream (15);
   - one or more distillation column(s) (16, 17) for distilling the crude methanol stream (15) to give a refined MeOH product (19, 22);

   wherein said one or more indirect heat exchanger(s) (7, 8, 32, 33) provide a heat input to said one or more distillation column(s) (16, 17), and/or to said MeOH synthesis loop (14), and/or to said electrolysis unit (4).

13. The plant (1) according to claim 12, wherein said one or more distillation column(s) (16, 17) is at least a topping column (16) and/or at least a refining column (17) comprising at least a bottom boiler; said bottom boiler being said one or more indirect heat exchanger(s) (7, 8), or being in a heat-exchanging relationship with said one or more indirect heat exchanger(s) (7, 8), for providing said heat input to said topping column (16) and/or refining column (17).

14. The plant (1) according to any of claims 12-13, comprising at least one indirect heat exchanger (8) in a heat-exchanging relationship with said cathode-side stream (5) and at least one indirect heat exchanger (7) in a heat-exchanging relationship with

**EP 4 686 717 A1**

said anode-side stream (6).

15. The plant (1) according to any of claims 12-14, wherein said electrolysis unit (4) comprises solid-oxide electrolysis cells (SOECs);
preferably said plant (1) comprising a renewable energy source (25), e.g. one or more photovoltaic panel(s) and/or wind turbine(s) and/or water turbine(s), powering said electrolysis unit (4) or solid-oxide electrolysis cells (SOECs).

Fig. 1.A

EP 4 686 717 A1

Fig. 1.B

Fig. 2

Fig. 3

Fig. 4

Fig. 5

EP 4 686 717 A1

Europäisches
Patentamt
European
Patent Office
Office européen
des brevets

# EUROPEAN SEARCH REPORT

**Application Number**

EP 24 19 1864

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 345 086 A1 (LINDE GMBH [DE]; UNIV MUENCHEN TECH [DE]) 3 April 2024 (2024-04-03) | 1-10, 12-14 | INV.<br>C07C29/80<br>C07C29/151 |
| A | * claims 1-15; figures 1-3 *<br>* paragraph [0014] *<br>* paragraph [0035] - paragraph [0037] *<br>* paragraph [0045] - paragraph [0059] *<br>----- | 11 | C07C31/04<br>C25B1/04<br>C25B1/23<br>C01B13/02 |
| X | WO 2023/217704 A1 (TOPSOE AS [DK]) 16 November 2023 (2023-11-16) | 1,2,10, 11,15 | |
| A | * claims 1-16; figures 1-2 *<br>* page 17, line 32 - page 19, line 5 *<br>----- | 3-9, 12-14 | |

| TECHNICAL FIELDS SEARCHED (IPC) |
|---|
| C07C<br>C25B<br>C01C<br>C01B |

The present search report has been drawn up for all claims

| Place of search | Date of completion of the search | Examiner |
|---|---|---|
| The Hague | 17 February 2025 | Seitner, Irmgard |

EPO FORM 1503 03.82 (P04C01)

## ANNEX TO THE EUROPEAN SEARCH REPORT
## ON EUROPEAN PATENT APPLICATION NO.

EP 24 19 1864

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2025

| Patent document cited in search report | | Publication date | Patent family member(s) | | Publication date |
|---|---|---|---|---|---|
| EP 4345086 | A1 | 03-04-2024 | EP | 4345086 A1 | 03-04-2024 |
| | | | WO | 2024068047 A1 | 04-04-2024 |
| WO 2023217704 | A1 | 16-11-2023 | AU | 2023266684 A1 | 10-10-2024 |
| | | | CN | 119095792 A | 06-12-2024 |
| | | | DK | 202200442 A1 | 13-02-2024 |
| | | | KR | 20250009423 A | 17-01-2025 |
| | | | WO | 2023217704 A1 | 16-11-2023 |

EPO FORM P0459

For more details about this annex : see Official Journal of the European Patent Office, No. 12/82